# EUROPÄISCHE PATENTSCHRIFT

(11) **EP 2 534 483 B1**
(45) Veröffentlichungstag und Bekanntmachung des Hinweises auf die Patenterteilung: **14.09.2016**
(21) Anmeldenummer: 11706460.0
(22) Anmeldetag: 09.02.2011
(51) Int. Cl.: G01N 33/50

(54) **TESTANORDNUNG**
TEST ARRANGEMENT
DISPOSITIF DE TEST

(30) Priorität: 10.02.2010 AT 1842010
(43) Veröffentlichungstag der Anmeldung: 19.12.2012
(73) Patentinhaber: Sigl, Eva, 8664 Gross Veitsch (AT)
(72) Erfinder: GÜBITZ, Georg, 8047 Hart bei Graz (AT); SIGL, Eva, 8664 Gross Veitsch (AT); HASMANN, Andrea, 8010 Graz (AT); SCHRÖDER, Marc, 9753 Heinerscheid (LU); SCHNEIDER, Konstantin, 8074 Raaba (AT); ROLLETT, Alexandra, 8010 Graz (AT); KAUFMANN, Franz, 79106 Freiburg (DE); HAFNER, Andreas, 4460 Gelterkinden BL (CH)
(74) Vertreter: Sonn & Partner Patentanwälte
(86) Internationale Anmeldenummer: PCT/AT2011/000074
(87) Internationale Veröffentlichungsnummer: WO 2011/097664

(56) Entgegenhaltungen:
- EP-A2- 0 261 931
- WO-A1-2007/065423
- WO-A2-2007/139854
- KIM ET AL: "Micropatterning of proteins on the surface of three-dimensional poly(ethylene glycol) hydrogel microstructures", ANALYTICA CHIMICA ACTA, ELSEVIER, AMSTERDAM, NL, Bd. 609, Nr. 1, 3. Januar 2008 (2008-01-03), Seiten 59-65, XP022450627, ISSN: 0003-2670, DOI: 10.1016/J.ACA.2007.12.024
- SALINAS-CASTILLO A ET AL: "Immobilization of a trienzymatic system in a sol-gel matrix: A new fluorescent biosensor for xanthine", BIOSENSORS AND BIOELECTRONICS, ELSEVIER BV, NL, Bd. 24, Nr. 4, 1. Dezember 2008 (2008-12-01), Seiten 1053-1056, XP025535198, ISSN: 0956-5663, DOI: 10.1016/J.BIOS.2008.07.052 [gefunden am 2008-08-03]
- K Schneider ET AL: "Trigger enzymes for pectin based bioresponsive polymers", XVIth International Conference on Bioencapsulation, 4 September 2008 (2008-09-04), XP055222948, Retrieved from the Internet: URL:http://impascience.eu/bioencapsulation /340_contribution_texts/2008-09-04_P76.pdf [retrieved on 2015-10-22]

## Beschreibung

Die vorliegende Erfindung betrifft eine Anordnung, welche geeignet ist, u.a. Mikroorganismen in einer Probe zu detektieren.

Der Nachweis von Mikroorganismen im Allgemeinen und die Identifizierung der nachgewiesenen Mikroorganismen in einer Probe ist in vielen Bereichen von erheblicher Wichtigkeit. So können beispielsweise Mikroorganismen für den Verderb oder die kurze Haltbarkeit von Lebensmitteln verantwortlich sein. Ferner spielen Mikroorganismen auch eine wichtige Rolle bei vielen Erkrankungen (z.B. Entzündungen) des Menschen und der Tiere. Es ist daher von großer Wichtigkeit das Vorhandensein von Mikroorganismen rechtzeitig zu erkennen, um entsprechende Maßnahmen zu setzen.

Im Stand der Technik sind verschiedenste Systeme und Verfahren beschrieben, die geeignet sind Mikroorganismen in einer Probe zu bestimmen. Diese Systeme können teilweise auch dazu eingesetzt werden spezielle Substanzen (z.B. Proteine wie Enzyme) in einer Probe nachzuweisen.

Zur Bestimmung mikrobieller Kontaminationen, beispielsweise, können Systeme eingesetzt werden die simple Adsorption erfordern. So wird zum Beispiel in der US 2001/017270 A1 eine Goldoberfläche mit immobilisierten Kohlenhydraten oder einem entsprechenden Derivat beschrieben, wobei die Bindung von Proteinen, Viren oder Bakterienzellen ein detektierbares Signal bewirkt.

In anderen Systemen werden andere äußere Einflüsse, wie zum Beispiel pH-Wert, ionische Stärke und Polarität zunutze gemacht. Eine durch die Anwesenheit von Mikroorganismen bedingte Änderung dieser Parameter kann z.B. zu einer Farbentwicklung oder - änderung führen.

In der WO 2006/065350 A2 erfolgt die Detektion von Mikroorganismen mittels eines Farbstoffes, welcher einen sichtbaren Farbwechsel bei Anwesenheit eines oder mehrerer Mikroorganismen zeigt. Dieser Farbwechsel basiert auf Unterschieden in der Polarität des Lösungsmittels bzw. Säure-Base-Reaktionen, Redox-Reaktionen, welche durch die Wechselwirkung zwischen Mikroorganismus und Sensor entstehen.

Ein weiteres, sehr einfaches System basiert auf der Detektion einer pH-Wert-Verschiebung während der mikrobiellen Kontamination (IE 20060034).

In der WO 98/010556 A1 wird unter anderem ein Verfahren geoffenbart, mit dessen Hilfe Moleküle an Bindungsstellen innerhalb eines Geltröpfchens gebunden werden können. Dabei werden Moleküle, welche von innerhalb des Geltröpfchens befindlichen Zellen sekretiert werden, an die freien Bindungsstellen der Geltröpfchen gebunden.

In der WO 03/033691 A1 wird ein Mikrochip geoffenbart, mit dessen Hilfe die antibakterielle Aktivität von Antibiotika getestet werden kann. Bei der Herstellung eines derartigen Chips werden prokaryotische und eukaryotische Zellen in einem Gel durch dessen Polymerisierung immobilisiert.

In der US 5,366,881 A werden polymerisierbare Lipide und Mischungen davon geoffenbart, in welche Wirkstoffe, Enzyme, fluoreszierende Substanzen und dgl. eingebracht werden können. Durch Stimulantien von außen, wie pH-Änderung oder Ionen, werden die eingekapselten Substanzen freigesetzt.

In der US 2006/0233854 A wird eine Matrix geoffenbart, welche aus einem Protein-"Backbone" besteht, welches mit Polyethylenglykol quervernetzt ist. Diese Matrix eignet sich insbesondere zur Gewebsregeneration. Die Matrix kann dabei zusätzlich einen biologisch aktiven Wirkstoff umfassen, der durch den Abbau der Matrix am Wirkort an das umliegenden Gewebe abgegeben wird. Um zu testen inwieweit diese Matrix den darin enthaltenen biologischen aktiven Wirkstoff freigibt, wurden gefärbte Fibrinogen-Fragmente in die Matrix aufgenommen.

Yan et al. (Biosens Bioelectron. 24 (8) (2009):2604-2610) beschreibt Biosensoren, die geeignet sind Wasserstoffperoxid in einer Probe zu bestimmen. Diese Biosensoren umfassen ein Hydrogel auf Polyethylenglykolbasis, welche zusätzlich mit Meerrettichperoxidase versetzt ist. Um schlussendlich am Biosensor direkt die Anwesenheit von Wasserstoffperoxid in einer Probe zu bestimmen, wird zusätzlich noch ein Substrat der Meerrettichperoxidase, nämlich Amplex Red, zugesetzt.

Ulijn et al. (Materials Today 10 (4) (2007):40-48) befassen sich mit bioresponsiven Hydrogelen. Darin werden verschiedenste Hydrogele diskutiert die je nach Mittel mit denen sie in Kontakt gebracht werden unterschiedliche Eigenschaften aufweisen.

Die US 2008/0057534 A1 beschreibt die visuelle Detektion einer mikrobiellen Kontamination durch den Abbau einer Deckschicht bestehend aus mikroben-sensitiven Farbstoffen. Diese mikroben-sensitiven Farbstoffe verändern bei Anwesenheit von Mikroben in der Probe die Farbe, so dass die Farbe entweder verloren geht oder ein Farbwechsel erfolgt. Die Farbstoffe können auf jede beliebige Unterlage aufgebracht werden. Eine gewisse, wenn auch sehr eingeschränkte, Selektivität wird durch die Art der Farbstoffe möglich.

Neben den Änderungen der unmittelbaren Umgebung können auch sekretierte Moleküle wie zum Beispiel Metabolite oder Enzyme zur Detektion von Mikroorganismen herangezogen werden. So beschreibt die EP 0 347 771, beispielsweise, ein Verfahren zur Charakterisierung von Enzymen verschiedener Bakterien, welche häufig in Blut und anderen Körperflüssigkeiten vorkommen. Sechsundvierzig verschiedene fluoreszierende Substrate können in einem derartigen Test verwendet werden. Anhand der Vielzahl an unterschiedlich fluoreszierenden Substrate und enzymatischen Reaktionsprofilen können mit diesem Verfahren unterschiedliche Mikroorganismen identifiziert werden.

Derartige Systeme können, z.B. in einem System wie in der US 2004/0018641 A1 beschrieben, zum Einsatz kommen. Durch Erscheinen oder Verschwinden von computerlesbaren Barcodes kann eine im Lebensmittelbereich vorhandene Kontamination aufgezeigt werden. Die Indikation beruht auf dem Nachweis von Gasen, Temperatur- und pH-Wert-Unterschieden, sowie Toxinen oder anderer Metaboliten aus Bakterien.

Auf der XVIth International Conference on Bioencapsulation, Dublin, Irland (4.-6. September 2008) präsentierten K. Schneider et al. Trigger-Enzyme für Pektin-basierte bioresponsive Polymere. In der der Präsentation zu Grunde liegenden Studie wurde eine Auswahl von Mikroorganismen im Hinblick auf deren Pectinase-Aktivität (d.h. Trigger-Enzym-Aktivität) durchrastert. Eine Matrix umfassend ein freisetzbares Enzym, das in der Lage ist ein farbänderndes Substrat umzusetzen, wie in Anspruch 1 definiert, wird darin jedoch weder offenbart noch nahegelegt.

Neben den visuell sichtbaren Farbstoffen kann auch Fluoreszenz herangezogen werden. Sehr oft werden hier Systeme basierend auf einer PCR Methode genutzt. Das beispielsweise in der US 2007/0122831 A1 beschriebene System nutzt die unterschiedlichen DNA Sequenzen verschiedener Mikroorganismen für die Detektion von mikrobieller Kontamination und zur Identifikation deren Ursprungs in wässrigen Proben.

Die US 6,297,059 B1 beschreibt ein Verfahren zur Messung mikrobieller Kontamination mittels Fluoreszenz aufgebaut auf eine flüssige Zweischichtmembran. Durch das Ansprechen mehrwertiger oder polyvalenter Zielbiomoleküle werden pro Molekül zwei oder mehrere Fluoreszenzquencher bzw. eine Übertragung der Extinktionsenergie auf einen Fluoreszenzakzeptor ausgelöst und somit eine Signalverstärkung erzielt.

Anordnungen und Vorrichtungen der oben beschriebenen Art weisen eine Reihe von Nachteilen auf. Einer der wesentlichsten Nachteile vieler der oben beschriebenen Systeme ist deren Trägheit und geringe Nachweisempfindlichkeit, da die Reaktionen in diesen Vorrichtungen, welche durch unterschiedliche Substanzen ("Trigger") ausgelöst werden, nicht verstärkt werden und dies nur langsam zu einer Signalgebung führt.

Weiters ist in vielen Fällen keine Schutzschicht vorhanden, sodass das System nur begrenzt stabil ist. Einfache Anzeigensysteme basierend auf Adsorption oder Verschiebung des pH-Wertes besitzen sehr geringe Selektivität und eine hohe Empfindlichkeit gegenüber äußeren Einflüssen. Alternativ hierzu können Systeme basierend auf Antikörpern verwendet werden. Diese haben jedoch den Nachteil hoher Kosten und den Bedarf an entsprechenden Instrumenten.

Es ist somit eine Aufgabe der vorliegenden Erfindung Vorrichtungen bzw. Anordnungen zur Verfügung zu stellen, welche die oben genannten Nachteile des Standes der Technik überwinden.

Die vorliegende Erfindung betrifft eine Anordnung umfassend einen festen Träger und eine an dem festen Träger angeordnete Matrix umfassend mindestens ein enzymatisch umsetzbares oder modifizierbares Molekül, vorzugsweise Polymer oder Oligomer, welche Matrix mindestens ein durch Umsetzung oder Modifikation des Moleküls, vorzugsweise Polymers oder Oligomers, freisetzbares Enzym umfasst, das in der Lage ist zumindest ein in der Matrix und/oder am festen Träger befindliches farberzeugendes Substrat umzusetzen.

Mit der erfindungsgemäßen Anordnung wird ein robustes System zur Verfügung gestellt, mit dessen Hilfe in einer Probe vorhandene Substanzen, insbesondere Enzyme, welche beispielsweise von Mikroorganismen sekretiert werden oder von anderen Quellen, wie beispielsweise dem Immunsystem stammen, zuverlässig detektiert werden können.

Die erfindungsgemäße Anordnung umfasst neben einem festen Träger und einer optionalen diesem gegenüberliegenden semipermeablen Membran eine Matrix, welche auf dem festen Träger bzw. gegebenenfalls zwischen dem festen Träger und der semipermeablen Membran angeordnet ist. Die Matrix umfasst mindestens ein Molekül, vorzugsweise Polymer, welches durch in der Probe vorhandene Substanzen (z.B. Enzyme), abgebaut, umgesetzt bzw. modifiziert werden kann. Durch den Abbau, die Umsetzung bzw. der Modifizierung derartiger Moleküle bzw. Polymere in der Matrix verändern sich deren Eigenschaften. Insbesondere kann die Umsetzung bzw. Modifikation des Moleküls bzw. Polymers oder Oligomers dazu führen, dass sich deren strukturellen Eigenschaften ändern, was zu einer Reduktion des Vernetzungsgrades bzw. der Viskosität, zur Änderung der Poren im Polymer oder zu einem Abbau der Polymerketten führen kann. Durch diese Modifikationen wird das in der Matrix befindliche freisetzbare Enzym mobiler und kann sich innerhalb der Matrix, insbesondere im Zwischenraum zwischen festem Träger und semipermeabler Membran, freier bewegen bzw. diffundieren. Durch die erhöhte Mobilität des Enzyms innerhalb der Anordnung wird diesem ermöglicht ein in der Matrix und/oder am festen Träger befindliches farberzeugendes Substrat umzusetzen bzw. freizusetzen und/oder das in der Matrix befindliche Molekül bzw. Polymer abzubauen.

Durch das Vorsehen von Enzymen in der erfindungsgemäßen Matrix, welches das Molekül bzw. Polymer in der Matrix umsetzt, abbaut oder modifiziert, wird die Mobilität der Enzyme in der Matrix vergrößert, sobald das Molekül bzw. Polymer durch extrinsische Faktoren aus der Probe abgebaut wird. Es kommt somit zu einer Farbreaktion und gegebenenfalls zu einer Verstärkung der Detektionsreaktion, da die Freisetzung der Molekül- bzw. Polymermodifizierenden bzw. -umsetzenden Enzyme in der Matrix, falls vorhanden, den Abbau des Moleküls bzw. Polymers verstärkt und beschleunigt. Dadurch ist es erstmals möglich geringste Konzentrationen solcher Stoffe bzw. Mikroorganismen in einer Probe nachzuweisen.

In der Matrix bzw. im Polymer können neben den Enzymen, die ein farberzeugendes Substrat umsetzen, auch Enzyme enthalten sein, die die Modifikation, Umsetzung oder den Abbau des Polymers unterstützen. Diese Enzyme sind entweder in das Polymer eingelagert oder an das Polymer über kovalente Bindungen gebunden. Im Zuge des Abbaus des Polymers werden diese Enzyme mobilisiert und können mit deren Substrat, welches am festen Träger gebunden ist oder sich im Polymer befindet, reagieren und eine Farbreaktion (z.B. Bildung von Farbe oder Änderung der Farbe) hervorrufen.

Ein derartiges System eignet sich beispielsweise um bereits geringste Mengen an Mikroorganismen, wie z.B. Bakterien oder Pilze, in einer Probe nachzuweisen. Dabei durchdringt ein von einem kontaminierenden Bakterium oder Pilz sekretiertes Enzym die schützende, semipermeable Membran, baut eine auf einem Trägermaterial aufgebrachte Matrix (z.B. ein Polysaccharid, ein Enzym oder Kombinationen davon, mit oder ohne Quervernetzung) ab und leitet somit die Freisetzung des Enzyms ein, welches als enzymatisches Verstärkungssystem, die Freisetzung beschleunigt und/oder die Detektion einer mikrobiellen Kontamination erst ermöglicht.

Es gibt eine Vielzahl von Möglichkeiten um das Polymer in der Matrix abzubauen, wie z.B. chemische, physikalische sowie biochemische. Beim chemischen Abbau wie z.B. einer Änderung des pH-Wertes, der ionischen Stärke oder chemischer Reagenzien werden die Wechselwirkungen zwischen den Polymerketten untereinander oder der Ketten mit den Lösungsmitteln auf molekularer Ebene verändert. Bei der physikalischen Stimulation (z.B. Temperatur, elektromagnetisches Feld und mechanischer Stress) erfolgt eine Veränderung molekularer Wechselwirkungen. Generell können biosensible Polymere und Mikrokapseln einfach auf pH-Wert-Änderungen in verschiedenen Umgebungen reagieren (Khayat, Int. J. Pharma 2006 317: 175-186; Li and Szoka Pharmaceutical Res. 2007 24:438-449; Nagareskar J. Biomed. Mater. Res. 2002 62: 195-203).

Bioresponsives Verhalten wird Bauteilen durch eine Beschichtung mit Biomaterialien, welche durch extrazelluläre Enzyme abgebaut werden können, verliehen. Extrazelluläre mikrobielle Enzyme können als sog. Trigger fungieren und somit die Freisetzung von im Polymer eingeschlossenen und/oder gebundenen aktiven Enzymen initiieren. Dabei definiert die Art des Polymers (Polypeptid, Polysaccharid, synthetisches Polymer...) die Art der benötigten Trigger-Enzyme. Mikroorganismen produzieren eine Vielzahl extrazellulärer Enzyme, die sie an die Umgebung abgeben um neben vielen anderen Funktionen, z.B. Nahrungsbestandteile, aufzuschließen um diese in die Zelle aufnehmen zu können. Extrazelluläre Enzyme dienen somit unter anderem dem Abbau von großen Molekülen und Polymeren. Die Hydrolyse einer Vielzahl von Biopolymeren wird von Hydrolasen katalysiert, die je nach Substrat in Lipasen, Proteasen, Esterasen, Glycosidasen, u.a. eingeteilt werden können.

Der Begriff "Polypeptid", wie hier verwendet, umfasst Polypeptide mit einer Mindestgröße von 50 Aminosäuren und schließt somit auch Proteine ein.

Ein "durch Abbau des Polymers freisetzbares Enzym" (Polypeptid) ist ein Enzym, welches in das Polymer der Matrix eingelagert ist und aufgrund der Quervernetzung des Polymers nicht diffundieren kann oder welches an das Polymer durch kovalente und/oder nicht kovalente Bindungen gebunden ist. In beiden Fällen sind die Enzyme vor Abbau des Polymers im Wesentlichen im Polymer immobilisiert bzw. nur beschränkt beweglich. Durch den Abbau, die Modifikation bzw. Umsetzung des Polymers werden dessen langkettige bzw. quervernetzte Ketten verkürzt bzw. gespalten, sodass die Enzyme in der Matrix freigesetzt, d.h. beweglicher, werden und darin herumdiffundieren können.

"Semipermeable Membran", wie hier verwendet, bezieht sich auf Membranen, die einen Zugang der Trigger bzw. Enzyme von außen in die erfindungsgemäße Anordnung erlaubt, aber die Diffusion der in der Matrix befindlichen Enzyme und anderer Matrixbestandteile nach außen verhindert.

Bei der Herstellung der erfindungsgemäßen Anordnung wird das im Polymer befindliche Enzym und das farberzeugende Substrat so gewählt, dass bei Inkontaktbringen beider Stoffe das Substrat umgesetzt wird und es dadurch zur Farbbildung kommt.

Das farberzeugende Substrat wird durch im Polymer befindliche Enzyme umgesetzt, wodurch es zu einer Farbbildung oder Farbänderung kommt. So kann z.B. durch Abspaltung einer chemischen Gruppe aus dem Substratmolekül oder durch Änderung des Redoxzustandes eine Farbbildung erfolgen. Die Farbbildung oder - änderung kann auch durch enzymatische Abspaltungen in der Umgebungsmatrix erfolgen (z.B. durch Abspaltung eines Quenchers, Änderung des pH-Wertes durch Umesterung).

Weiters kann auch eine in-situ Farbstoffsynthese oder Farbstoffentfärbung durch die enzymatische Umsetzung eines oder mehreren Substraten stattfinden, z.B. Polymerisation oder Depolymerisation eines Phenols mittels Oxidoreduktase.

Das Indikatorsystem kann auch darin bestehen, dass das Verstärker-Enzym in der Lage ist, eine verdeckende Polymerschicht hydrolytisch abzubauen und somit die verdeckte Farbschicht in Erscheinung treten zu lassen.

In der folgenden Tabelle A sind besonders bevorzugte Kombinationen von Polymer (und dessen abbauendes Enzym), farbänderndes Enzym und dessen Substrat angeführt:

| **Polymer** | **Abbauendes Trigger Enzym** | **Eingeschlossenes Polypeptid (Verstärkerenzym) bzw. gebundener Farbstoff** | **Farbreaktion durch Umsatz von** |
|---|---|---|---|
| Pektin | Pektinase | PEG-Protease | Siloxan -N Succinyl Ala-Ala-Pro-Leu-p-Nitroanilid |
| Pektin | Pektinase | Protease | Siloxan -N Succinyl Ala-Ala-Pro-Leu-p-Nitroanilid |
| Pektin | Pektinase | PVA -Protease | Siloxan -N Succinyl Ala-Ala-Pro-Leu-p-Nitroanilid |
| Pektin | Pektinase | Laccase | Siloxan-Ferulasäure |
| Pektin | Pektinase | Laccase | Siloxan-Kaffeesäure |
| Pektin | Pektinase | Laccase | Siloxan 3,4, Dihydroxybenzoesäure |
| Pektin | Pektinase | PEG- Laccase | Siloxan-Ferulasäure |
| Pektin | Pektinase | PEG- Laccase | Siloxan-Kaffeesäure |
| Pektin | Pektinase | PEG -Laccase | Siloxan 3,4, Dihydroxybenzoesäure |
| Pektin | Pektinase | PEG Laccase | ABTS |
| Pektin/Alginat | Pektinase | PEG-Protease | Siloxan -N Succinyl Ala-Ala-Pro-Leu-p-Nitroanilid |
| Pektin/Alginat | Pektinase | Protease | Siloxan -N Succinyl Ala-Ala-Pro-Leu-p-Nitroanilide |
| Pektin/Alginat | Pektinase | PVA -Protease | Siloxan -N Succinyl Ala-Ala-Pro-Leu-p-Nitroanilide |
| Pektin/Alginat | Pektinase | Laccase | Siloxan-Ferulasäure |
| Pektin/Alginat | Pektinase | Laccase | Siloxan-Kaffeesäure |
| Pektin/Alginat | Pektinase | Laccase | Siloxan 3,4, Dihydroxybenzoesäure |
| Pektin/Alginat | Pektinase | PEG Laccase | Siloxan-Ferulasäure |
| Pektin/Alginat | Pektinase | PEG Laccase | Siloxan-Kaffeesäure |
| Pektin/Alginat | Pektinase | PEG Laccase | Siloxan 3,4, Dihydroxybenzoesäure |
| Pektin/Alginat | Pektinase | PEG Laccase | ABTS |
| Pektin/Alginat | Pektinase | Laccase | ABTS |
| Peptidoglycan/Alginat | Lysozym | Laccase | ABTS |
| Peptidoglycan/Alginat | Lysozym | PEG-Laccase | ABTS |
| Peptidoglycan/Alginat | Lysozym | PEG Laccase | Siloxan-Ferulasäure |
| Peptidoglycan/Alginat | Lysozym | PEG Laccase | Siloxan-Kaffesäure |
| Peptidoglycan/Alginat | Lysozym | PEG Laccase | Siloxan 3,4, Dihydroxybenzoesäure |
| Peptidoglycan/Alginat | Lysozym | PEG-Protease | Siloxan -N Succinyl Ala-Ala-Pro-Leu-p-Nitroanilide |
| Peptidoglycan/Alginat | Lysozym | Protease | Siloxan -N Succinyl Ala-Ala-Pro-Leu-p-Nitroanilide |
| Peptidoglycan/Alginat | Lysozym | PVA -Protease | Siloxan -N Succinyl Ala-Ala-Pro-Leu-p-Nitroanilide |
| Peptidoglycan/Agarose | Lysozym | Laccase | ABTS |
| Peptidoglycan/Agarose | Lysozym | PEG-Laccase | ABTS |
| Peptidoglycan/Agarose | Lysozym | PEG Laccase | Siloxan-Ferulasäure |
| Peptidoglycan/Agarose | Lysozym | PEG Laccase | Siloxan-Kaffeesäure |
| Amylopektin | Amylase | PEG Laccase | Siloxan 3,4, Dihydroxybenzoesäure |
| Peptidoglycan/Agarose | Lysozym | PEG-Protease | Siloxan -N Succinyl Ala-Ala-Pro-Leu-p-Nitroanilide |
| Peptidoglycan/Agarose | Lysozym | Protease | Siloxan -N Succinyl Ala-Ala-Pro-Leu-p-Nitroanilide |
| Peptidoglycan/Agarose | Lysozym | PVA -Protease | Silocxan -N Succinyl Ala-Ala-Pro-Leu-p-Nitroanilide |
| Elastin/Agarose | Elastase | PEG-Protease | Siloxan -N Succinyl Ala-Ala-Pro-Leu-p-Nitroanilid |
| Elastin/Agarose | Elastase | Protease | Silocxan -N Succinyl Ala-Ala-Pro-Leu-p-Nitroanilid |
| Elastin/Agarose | Elastase | PVA -Protease | Siloxan -N Succinyl Ala-Ala-Pro-Leu-p-Nitroanilide |
| Elastin/Agarose | Elastase | Laccase | Siloxan-Ferulasäure |
| Elastin/Agarose | Elastase | Laccase | Siloxan-Kaffeesäure |
| Elastin/Agarose | Elastase | Laccase | Siloxan 3,4, Dihydroxybenzoesäure |
| Elastin/Agarose | Elastase | PEG-Laccase | ABTS |
| Elastin/Agarose | Elastase | PEG Laccase | Siloxan-Ferulasäure |
| Elastin/Agarose | Elastase | PEG Laccase | Siloxan-Kaffesäure |
| Elastin/Agarose | Elastase | PEG Laccase | Siloxan 3,4, Dihydroxybenzoesäure |
| Silk/Agarose | Elastase | PEG-Protease | Siloxan -N Succinyl Ala-Ala-Pro-Leu-p-Nitroanilide |
| Silk/Agarose | Elastase | Protease | Siloxan -N Succinyl Ala-Ala-Pro-Leu-p-Nitroanilide |
| Silk/Agarose | Elastase | PVA -Protease | Silocxan -N Succinyl Ala-Ala-Pro-Leu-p-Nitroanilide |
| Silk/Agarose | Elastase | Laccase | Siloxan-Ferulasäure |
| Silk/Agarose | Elastase | Laccase | Siloxan-Kaffesäure |
| Silk/Agarose | Elastase | Laccase | Siloxan 3,4, Dihydroxybenzoesäure |
| Silk/Agarose | Elastase | PEG-Laccase | ABTS |
| Silk/Agarose | Elastase | PEG Laccase | Siloxan-Ferulasäure |
| Silk/Agarose | Elastase | PEG Laccase | Siloxan-Kaffeesäure |
| Silk/Agarose | Elastase | PEG Laccase | Siloxan 3,4, Dihydroxybenzoesäure |
| Chitosan /Agarose | Lysozym | PEG-Laccase | ABTS |
| Chitosan /Agarose | Lysozym | PEG Laccase | Siloxan-Ferulasäure |
| Chitosan /Agarose | Lysozym | PEG Laccase | Siloxan-Kaffesäure |
| Chitosan /Agarose | Amylase | PEG Laccase | Siloxan 3,4, Dihydroxybenzoesäure |
| Chitosan /Agarose | Lysozym | PEG-Protease | Siloxan -N Succinyl Ala-Ala-Pro-Leu-p-Nitroanilide |
| Chitosan /Agarose | Lysozym | Protease | Siloxan -N Succinyl Ala-Ala-Pro-Leu-p-Nitroanilide |
| Chitosan /Agarose | Lysozym | PVA -Protease | Siloxan -N Succinyl Ala-Ala-Pro-Leu-p-Nitroanilide |
| Gelatine/Agarose | Gelatinase | Laccase | ABTS |
| Gelatine/Agarose | Gelatinase | PEG-Laccase | ABTS |
| Gelatine/Agarose | Gelatinase | PEG Laccase | Siloxan-Ferulasäure |
| Gelatine/Agarose | Gelatinase | PEG Laccase | Siloxan-Kaffesäure |
| Gelatine/Agarose | Gelatinase | PEG Laccase | Siloxan 3,4,Dihydroxybenz oesäure |
| Gelatine/Agarose | Gelatinase | PEG-Protease | Siloxan -N Succinyl Ala-Ala-Pro-Leu-p-Nitroanilide |
| Gelatine/Agarose | Gelatinase | Protease | Siloxan -N Succinyl Ala-Ala-Pro-Leu-p-Nitroanilide |
| Gelatine/Agarose | Gelatinase | PVA -Protease | Siloxan -N Succinyl Ala-Ala-Pro-Leu-p-Nitroanilide |
| Gelatine/Alginat | Gelatinase | Laccase | ABTS |
| Gelatine/Alginat | Gelatinase | PEG-Laccase | ABTS |
| Gelatine/Alginat | Gelatinase | PEG Laccase | Siloxan-Ferulasäure |
| Gelatine/Alginat | Gelatinase | PEG Laccase | Siloxan-Kaffesäure |
| Gelatine/Alginat | Gelatinase | PEG Laccase | Siloxan 3,4,Dihydroxybenz oesäure |
| Gelatine/Alginat | Gelatinase | PEG-Protease | Siloxan -N Succinyl Ala-Ala-Pro-Leu-p-Nitroanilide |
| Gelatine/Alginat | Gelatinase | Protease | Siloxan -N Succinyl Ala-Ala-Pro-Leu-p-Nitroanilide |
| Xylan | Xylanase | Laccase | ABTS |
| Xylan | Xylanase | Xylan | ABTS |
| Xylan | Xylanase | PEG Laccase | Siloxan-Ferulasäure |
| Xylan | Xylanase | PEG Laccase | Siloxan-Kaffesäure |
| Xylan | Xylanase | PEG Laccase | Siloxan 3,4,Dihydroxybenz oesäure |
| Xylan | Xylanase | PEG-Protease | Siloxan -N Succinyl Ala-Ala-Pro-Leu-p-Nitroanilide |
| Xylan | Xylanase | Protease | Siloxan -N Succinyl Ala-Ala-Pro-Leu-p-Nitroanilide |
| Cellulose | Cellulase | Laccase | ABTS |
| Cellulose | Cellulase | Xylan | ABTS |
| Cellulose | Cellulase | PEG Laccase | Siloxan-Ferulasäure |
| Cellulose | Cellulase | PEG Laccase | Siloxan-Kaffesäure |
| Cellulose | Cellulase | PEG Laccase | Siloxan 3,4,Dihydroxybenz oesäure |
| Cellulose | Cellulase | PEG-Protease | Siloxan -N Succinyl Ala-Ala-Pro-Leu-p-Nitroanilide |
| Cellulose | Cellulase | Protease | Siloxan -N Succinyl Ala-Ala-Pro-Leu-p-Nitroanilide |
| Dextran | Amylase | Laccase | ABTS |
| Dextran | Amylase | Xylan | ABTS |
| Dextran | Amylase | PEG Laccase | Siloxan-Ferulasäure |
| Dextran | Amylase | PEG Laccase | Siloxan-Kaffesäure |
| Dextran | Amylase | PEG Laccase | Siloxan 3,4,Dihydroxybenz oesäure |
| Dextran | Amylase | PEG-Protease | Siloxan -N Succinyl Ala-Ala-Pro-Leu-p-Nitroanilide |
| Dextran | Amylase | Protease | Siloxan -N Succinyl Ala-Ala-Pro-Leu-p-Nitroanilide |

Als Verstärker-Enzyme für die Amplifikation der Anzeige können Oxidoreduktasen wie Laccasen oder Peroxidasen,Proteasen, Lipasen, Esterasen, Peptidasen, etc. eingesetzt werden.

Gemäß einer bevorzugten Ausführungsform der vorliegenden Erfindung ist die Matrix als Schicht, in Form von Kapseln oder als Hydrogel, auf dem festen Träger angeordnet.

Die Matrix zwischen dem festen Träger und der semipermeablen Membran kann unterschiedliche Formen aufweisen. Die Matrix kann beispielsweise als Schicht oder Kapseln aufgebracht werden. Die Verwendung von Kapseln bzw. mehrerer Schichten (mindestens zwei, drei oder vier) hat den Vorteil, dass die Kapseln und Schichten unterschiedliche Zusammensetzungen aufweisen können. So ist es möglich eine Kapselart bzw. eine Schicht zur Verfügung zu stellen, die Enzyme umfassen, die in der Lage sind das Polymer der Matrix abzubauen. Andere Kapseln bzw. Schichten können hingegen Enzyme umfassen, die in der Lage sind farberzeugende Substrate umzusetzen. Auch die Polymerzusammensetzung der verschiedenen Kapseln bzw. Schichten können variieren.

Gemäß einer bevorzugten Ausführungsform der vorliegenden Erfindung kann die Matrix ein abbaubares, modifizierbares Molekül bzw. Polymer umfassen, welches durch Enzyme abgebaut bzw. modifiziert werden kann. Selbstverständlich können aber auch Moleküle bzw. Polymere eingesetzt werden, die im Zuge einer pH-Wert-Änderung oder durch Änderung der Ionenstärke abbaubar sind.

Das enzymatisch zersetzbare, umsetzbare oder modifizierbare bzw. abbaubare Polymer ist vorzugsweise ein Polysaccharid, Polypeptid, Polyester, Polyamid oder eine Kombination davon.

Das abbaubare bzw. modifizierbare Polymer kann, um den Abbau bzw. die Modifikation durch in der Probe vorhandenen Enzymen zu steuern, beispielsweise, verestert sein.

Gemäß einer bevorzugten Ausführungsform der vorliegenden Erfindung ist das Polysaccharid ausgewählt aus der Gruppe bestehend aus Pektin, Amylose, Amylopektin, Agarose, Alginat, Carraghenan, Chitin, Chitosan, Dextran, Glycogen, Guar, Johannisbrotkernmehl, Laevan, Pektin, Pollulan, Tamarindenkernmehl, Xanthan, Zellulose und Xylan.

Um eine weitere Variationsmöglichkeit zu besitzen besteht ebenfalls die Möglichkeit das Polymer mit funktionellen Seitenketten zu modifizieren. Diese Seitenketten sind in der Lage duch kovalentes Quervernetzen ein gegen Autohydrolyse stabiles Polymernetzwerk zu bilden. Die Abbaubarkeit durch die bereits beschriebenen Trigger Enzyme wird dadurch nicht beeinflusst. Möglichkeiten für derartige Seitenketten wären radikalisch polymerisierende Monomere wie Acrylate bzw. Methacrylate.

Polymere auf Proteinbasis, welche erfindungsgemäß eingesetzt werden können, können Seide und Elastin ähnliche Proteinpolymere sein.

Um eine verbesserte Spezifität der Polymere zu erreichen, können die Polymere mit spezifischen Sequenzen (z.B. Aminosäure-oder Oligosaccharidsequenzen) versehen werden, welche Spaltstellen für bestimmte Enzyme darstellen. Damit wird ermöglicht Polymere herzustellen, die in Kontakt mit den nachzuweisenden Substanzen (z.B. Enzymen) gespalten werden.

Gemäß einer weiteren bevorzugten Ausführungsform der vorliegenden Erfindung ist das abbaubare Polymer ausgewählt aus der Gruppe bestehend aus Gummiarabicum, Agar, Agarose, Maltodextrine, Alginsäure und deren Salze, insbesondere Natriumalginat oder Calciumalginat, Liposome, Fette, Cetylalkohol, Collagen, Chitosan, Peptidoglycan, Leithine, Gelatine, Albumin, Schellack, Polysaccaride, insbesondere Stärke oder Dextran, Cyclodextrine, Pektin, Carragenan und Wachse.

Gemäß einer bevorzugten Ausführungsform der vorliegenden Erfindung ist die polymerabbauende Verbindung ein Enzym.

Das Enzym welches in der Matrix umfasst ist, ist vorzugsweise ausgewählt aus der Gruppe bestehend aus den Gruppen der Hydrolasen und Oxidoreduktasen wie zum Beispiel Proteasen, Laccasen oder Peroxidasen.

Um die Mobilität der Enzyme im Polymer vor dessen Abbau einzuschränken, um den vorzeitigen Abbau des Polymers zu verhindern bzw. zu unterbinden und um zu verhindern, dass das farberzeugende Substrat vorzeitig umgesetzt wird, ist das durch die Umsetzung oder Modifikation bzw. den Abbau des Moleküls bzw. Polymers freisetzbare Enzym chemisch oder adsorptiv, z.B., an Polyvinylalkohol, Polyethylenglykol (PEG) oder Peptide gebunden oder dessen Molekulargewicht genetisch durch Fusion, z.B. mit Elastin oder anderen Peptiden oder Proteinen vergrößert. Durch die Bindung des Polypeptids an diese hochmolekularen Gruppen wird die Diffusion im Polymer im Wesentlichen verhindert.

Gemäß einer bevorzugten Ausführungsform der vorliegenden Erfindung ist das farberzeugende Substrat ausgewählt aus der Gruppe bestehend aus phenolischen Verbindungen und Azo-Farbstoffen wie Ferulasäure, Kaffeesäure, 3,4-Dihydroxybenzoesäure, Reactive Blue, Indigo Carmine, ABTS oder Guaiacol.

Die semipermeable Membran ist vorzugsweise ausgewählt aus der Gruppe bestehend aus Cellulosederivate, Polyamide, Polyacrylamide und Polyester.

Ein semipermeabler Charakter wird durch Modifikation der Oberfläche einer Seite erreicht die z.B. hydrophiler oder hydrophober gemacht wird. Dies kann durch z.B. durch chemische, physikalische (Plasma) oder enzymatische Behandlung erzielt werden (z.B. Guebitz,G.M.,Cavaco-Paulo,A., 2008. Trends in Biotechnology 26, 32-38).

Gemäß einer bevorzugten Ausführungsform der vorliegenden Erfindung umfasst der feste Träger ein Material, welches ausgewählt ist aus der Gruppe bestehend aus anorganischen Materialien, vorzugsweise Silicagel, Aluminium, Silizium oder Glas, organischen Materialien, vorzugsweise Polyester, Polystyrol, Polyamid, Polyacrylamid oder Polyvinylalkohol, oder Biopolymeren, vorzugsweise Papier.

Um die Bindung zwischen Trägermaterial und Matrix zu verbessern kann das Trägermaterial chemisch modifiziert werden. Bei der Modifikation werden funktionelle Gruppe auf die Oberfläche aufgebracht die anschließend mit Bestandteilen der Matrix kovalent quervernetzen können. Als Beispiel sei an dieser Stelle Trimethylsilyl methacrylate genannt. Wie an andere Stelle beschrieben können Trimethylsilyl Gruppen mit Hydroxygruppen an Oberflächen wie sie bei Glas aber auch bei andere Polymeren vorkommen kovalente Etherbrücken bilden. Die Metacrylgruppe kann im Anschluss mit Methacrylgruppen der Matrix quervernetzen.

Ein weiterer Aspekt der vorliegenden Erfindung betrifft die Verwendung einer erfindungsgemäßen Anordnung zur Bestimmung der Anwesenheit und/oder Charakterisierung von Zellen, vorzugsweise von Mikroorganismen, in einer Probe.

Die zu bestimmenden und/oder charakterisierenden Mikroorganismen sind ausgewählt aus der Gruppe bestehend aus Bakterien und Pilzen.

Die erfindungsgemäße Anordnung kann gemäß einem weiteren Aspekt zur Detektion mindestens eines Enzyms in einer Probe verwendet werden. Ein noch weiterer Aspekt der vorliegenden Erfindung betrifft die Verwendung einer erfindungsgemäßen Anordnung zur Detektion einer Wundinfektion durch Bestimmen des Vorhandenseins mindestens eines wund-spezifischen Enzyms.

Gemäß der vorliegenden Erfindung sind "wund-spezifische" Enzyme Enzyme, die üblicherweise bei Infektionen von Wunden auftreten. Beispielhafte Enzyme sind solche Enzyme, die von den die Infektion auslösenden Mikroorganismen sekretiert werden oder solche Enzyme, die vom Körper als Antwort auf eine Infektion ausgeschüttet werden.

Gemäß einer bevorzugten Ausführungsform der vorliegenden Erfindung ist das mindestens eine Enzym ausgewählt aus der Gruppe der Hydrolasen bestehend aus Amylase, Cellulase, Xylanase, Mannanase, Protease, Lysozym, Lipase und Esterase, Oxidase.

Die gegenständliche Erfindung wird ferner anhand der folgenden Figuren und Beispiele näher illustriert.
Fig. 1 zeigt die Freisetzung von Alizarinfarbstoff mittels verschiedenen Mikroorganismen nach 72 Stunden Inkubation (Beispiel 3).
Fig. 2 zeigt den Zeitverlauf der Freisetzung von Proteasen aus dem Biopolymer durch Einwirkung von Pektinasen (Beispiel 7).
Fig. 3 zeigt den Zeitverlauf der Freisetzung des Verstärkerenzyms Laccase aus dem Biopolymer durch Einwirkung eines Triggerenzyms (Pektinasen) (Beispiel 7).
Fig. 4 zeigt die Freisetzung von p-Nitroanilid des immobilisierten Substrats mittels kommerzieller Protease (Beispiel 8).
Fig. 5 zeigt den Farbumschlag immobilisierter Ferulasäure. Durch Einwirkung des Triggerenzyms (Pektinase) wird das Verstärkerenzym (Laccase) aus dem Biopolymer freigesetzt und induziert die Farbreaktion (Farbgebung durch Oxidation immobilisierter Ferulasäure) (Beispiel 10).
Fig. 6 zeigt die Umsetzung von ABTS (Diammonium-2,2'-azinobis-(3-ethylbenzthiazolin-6-sulfonsäure)) durch Laccase infolge deren Freisetzung aus einer Peptidoglycan Matrix nach Inkubation mit Lysozym (5000 U/mL) oder Puffer (Kontrolle) nach verschiedenen Zeitpunkten.

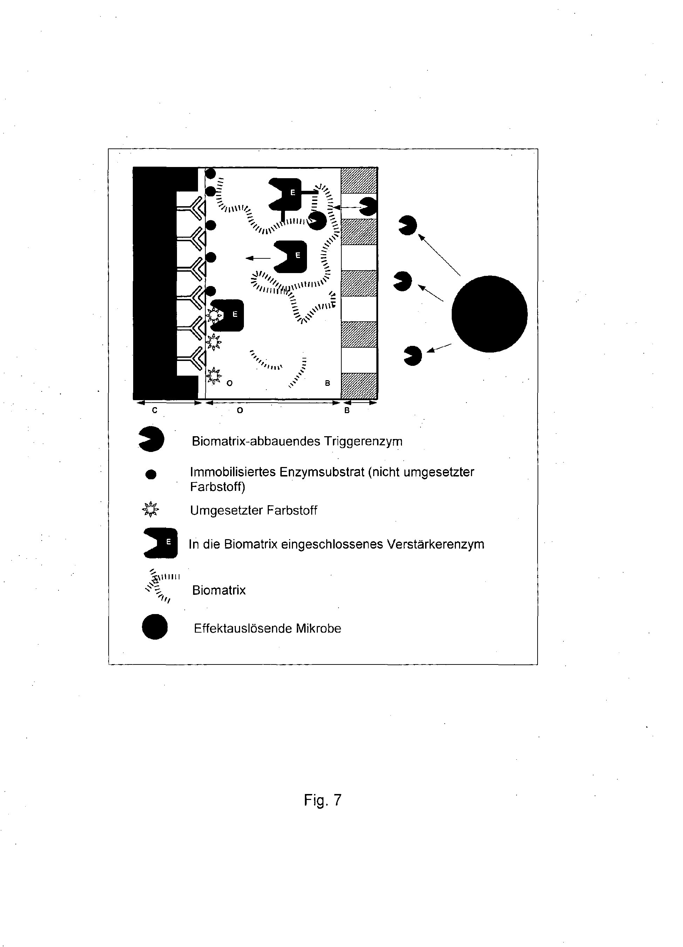

Fig. 7 zeigt ein erfindungsgemäßes bioresponsives System mit enzymatischer Verstärkungsreaktion zur kontrollierten Freisetzung und Sensorik.

### BEISPIELE

### Beispiel 1:

### Herstellung eines Biopolymers A

Eine 5%ige Lösung aus Pektin, gewonnen aus Citrusschalen mit einem Veresterungsgrad von 50-60%, wurde durch Auflösen in Wasser über Nacht bei 50°C hergestellt. Alternativ kann auch Pektin aus Apfelschalen mit einem Veresterungsgrad von 70-75% verwendet werden.

Im Vorfeld wurde das Polysaccharid mit verschiedenen Farbstoffen wie z.B. Alizarin, Cibacrom, Remazol, Viktoriablau oder anderen gefärbt. Dazu wurden 10g Pektin in Aceton mit 5mM Farbstoff suspendiert, über Nacht unter Rückfluss erhitzt und anschließend mehrere Male mit Aceton gewaschen. Die Pektinlösung wurde durch Eintropfen in eine 200mM CaCl₂ Lösung auspolymerisiert und die erhaltenen Pektinkugeln wurden mit Wasser gewaschen.

### Enzymatischer Abbau von Biopolymer A

1g (Feuchtmasse) des Biopolymers A wurde mit Alizarin gefärbt und in 10mL Puffer (50mM, pH6,0) für 24h bei Raumtemperatur unter leichtem Schütteln unter Verwendung verschiedener kommerziell erhältlicher Pektinasen inkubiert. Anschließend wurden die Überstände mit 1M NaOH auf pH14 eingestellt und die Adsorption bei 550nm mit Hilfe eines UV/VIS Photometers gemessen.

### Mikrobiologischer Abbau von Biopolymer A

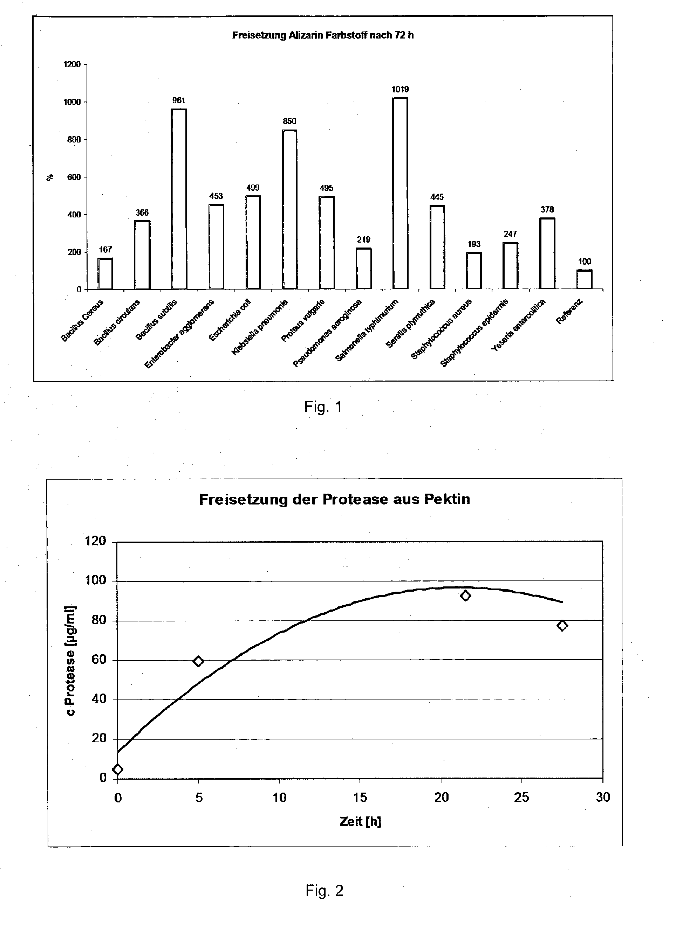

Unterschiedliche potentiell kontaminierende Mikroorganismen wurden mittels Vorkultur angezüchtet. Zu 100mL der Hauptkultur wurden jeweils 1g Feuchtmasse des mit Alizarin gefärbten Biopolymers A zugegeben und mit 100µL Vorkultur inokuliert. Die Inkubation wurde für 72 Stunden bei 33°C durchgeführt. Anschließend wurde die Biomasse abzentrifugiert und die Absorption des Überstandes bei geeigneter Wellenlänge gemessen (Fig. 1).

### Beispiel 2:

### Siloxanimmobilisierung des Substrats für das Verstärkerenzym

### Methode A

10g Kieselgel wurden in 30mL einer 3-9% Aminopropyltriethoxysilan in Ethanol (95%) für 4 Stunden bei 40°C gerührt. Anschließend wurde das aminierte Kieselgel abdekantiert, 3-mal mit 70% Ethanol gewaschen und im Exsikkator bis zur Gewichtskonstanz getrocknet.

50mg N-(3-Dimethylaminopropyl)-N-ethylcarbodiimid hydrochlorid, 5mg (1-Hydroxybenzotriazole hydrate) und 50mg Ferulasäure wurden in 20mL absolutem Ethanol gelöst. Anschließend wurde 5g aminiertes Kieselgel zugegeben und 30min gerührt. Nach Zentrifugation wurde das Kieselgel mit der gekoppelten Ferulasäure mit 70% Ethanol gewaschen und im Exsikkator bis zur Gewichtskonstanz getrocknet. Alternativ zur Ferulasäure kann Kaffeesäure, 3,4-Dihydroxybenzoesäure, oder Fast Blue RR etc. verwendet werden. Weiters können auch Proteasesubstrate wie z.B. N-Succinyl-Ala-Ala-Pro-Leu-p-nitroanilid, N-Succinyl-Ala-Ala-Pro-Val-p-nitroanilid oder L-Leucin-p-nitroanilid auf die gleiche Art und Weise immobilisiert werden.

### Methode B

10g Kieselgel wurde in 30mL einer 3-9% Mercaptopropyltriethoxysilan in Ethanol (95%) für 4 Stunden bei 40°C gerührt. Anschließend wurde das Kieselgel abdekantiert, 3-mal mit 2-Propanol gewaschen und im Exsikkator bis zur Gewichtskonstanz getrocknet. Zu 5g vorbehandeltem Kieselgel, suspendiert in 20mL Dichlormethan, werden 155mg Ferulasäure und 38mg Dimethylaminopyridin zugegeben. Zu der mit Eis auf 0°C gekühlten Reaktionsmischung wurden 165mg DCC zugegeben und über 3 Stunden gerührt. Dabei wurde die Temperatur auf Raumtemperatur (ca. 20°C) belassen. Anschließend wurde das feste, modifizierte Kieselgel abfiltriert, 3-mal mit Dichlormethan gewaschen und über Nacht im Exsikkator getrocknet.

### Beispiel 3:

### Herstellung des Biopolymers B

Pektin, gewonnen aus Citrusschalen mit einem Veresterungsgrad von 50-60%, wurde unter leichtem Erhitzen über Nacht in Wasser gelöst. Zum Pektin als Hauptbestandteil der bioresponsiven Matrix wurde Alginat in verschiedenen Konzentrationen (1-20%) beigemengt.

Vorzugsweise wurde eine Mischung bestehend aus 4,5g Pektin und 0,5g Natriumalginat in 100mL Wasser verwendet. Die Gelherstellung erfolgte durch Eintropfen der Pektin-Alginat-Lösung in eine vorgelegte 200mM CaCl₂ Lösung. Die erhaltenen Pektinkugeln wurden mit Wasser gewaschen.

### Beispiel 4:

### Inkorporation von Enzymen und Proteinen in Biopolymer B

Zu einer Mischung bestehend aus 4,5g Pektin und 0,5g Natriumalginat in 100mL Wasser wurden 5mL kommerziell erhältlicher Protease von Aspergillus oryzae zugegeben.

Nach dem Vermischen der Polysaccharidlösung mit dem Enzym wurde das Polymer in 200mM CaCl₂-Lösung eingetropft und ausgeliert. Die so gewonnenen Polymerkügelchen wurden abgesiebt, 3-mal mit 50mM Tris-HCl Puffer pH7,5 gewaschen und zu je 1g Feuchtmasse in Reaktionsgefäßen mit 5mL 50mM Tris-HCl Puffer pH7,5 portioniert.

Entsprechend können auch andere Enzyme wie z.B. Laccasen verschiedener Trametes sp. immobilisiert werden. Weiters können auch Proteine z.B. Casein, Collagen etc. entsprechend eingebaut werden.

### Beispiel 5:

### Freisetzung von Enzymen mittels Pektinasen

Als Testpolymer wurde das Biopolymer B (Beispiel 4) mit einer Protease von Aspergillus oryzae beladen.

Die so gewonnenen Polymerkugeln wurden abgesiebt, 3-mal mit 50mM Tris-HCl Puffer pH7,5 gewaschen und zu je 1g Feuchtmasse in Reaktionsgefäßen mit 5mL 50mM Tris-HCl Puffer pH7,5 portioniert. Der enzymatische Abbau wurde durch Zugabe einer kommerziellen Pektinase gestartet. Während der Inkubation bei Raumtemperatur und unter Schütteln wurden in bestimmten Zeitintervallen Proben aus dem Überstand genommen. Von diesen Proben wurden die Proteaseaktivitäten mittels Azocasein Assay und der Proteingehalt bestimmt (Fig. 2).

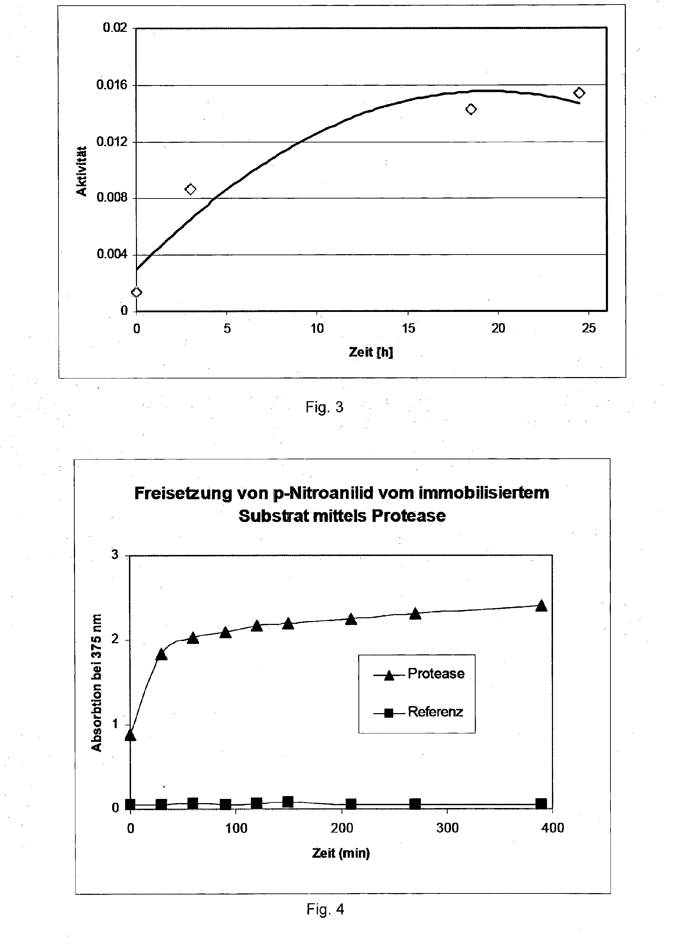

Alternativ dazu kann das Biopolymer mit jedem anderen Enzym, wie z.B. Laccasen, aus der Gruppe der Oxidoreduktasen beladen werden. Hier wird dann entsprechend die Aktivität der freigesetzten Laccase mittels ABTS bestimmt (Fig. 3).

### Beispiel 6:

### Enzymatische Signalgebung und deren Verstärkung mittels Proteasen

10mg Kieselgel mit immobilisiertem N-Succinyl-Ala-Ala-Pro-Leu-p-nitroanilid bzw. L-Leucin-p-nitroanilid oder N-Succinyl-Ala-Ala-Pro-Val-p-nitroanilid als Proteasesubstrate (Beispiel 2) wurden in 1300µL 50mM Tris-HCl Puffer pH8,3 suspendiert und mit kommerziell erhältlicher Protease von Aspergillus oryzae bzw. mit Wundflüssigkeit bei Raumtemperatur inkubiert. Die Aktivität wurde mittels UV-Absorption des abgespaltenen p-Nitroanilides im Überstand bei 375nm bzw. bei 405nm bestimmt (Fig. 4).

### Beispiel 7:

### Enzymatische Signalgebung und deren Verstärkung mittels Lacca sen

10mg Kieselgel mit immobilisierter Ferulasäure als Laccasesubstrat (Beispiel 2) bzw. mit immobilisiertem Fast Blue RR wurden in 1300µL 50mM Succinat Puffer pH4,5 suspendiert und mit Laccase vom Trametes hirsuta bzw. mit Wundflüssigkeit bei Raumtemperatur inkubiert. Die Aktivität wird anhand einer Farbmessung (gelb-orange) mittels Spectrophotometer bestimmt.

### Beispiel 8:

### Enzymatische Signalgebung und deren Verstärkung durch Freisetzung von Laccasen mittels Pektinasen

Als Testpolymer diente das Biopolymer aus Beispiel 4 beladen mit Laccasen verschiedener Trametes sp.

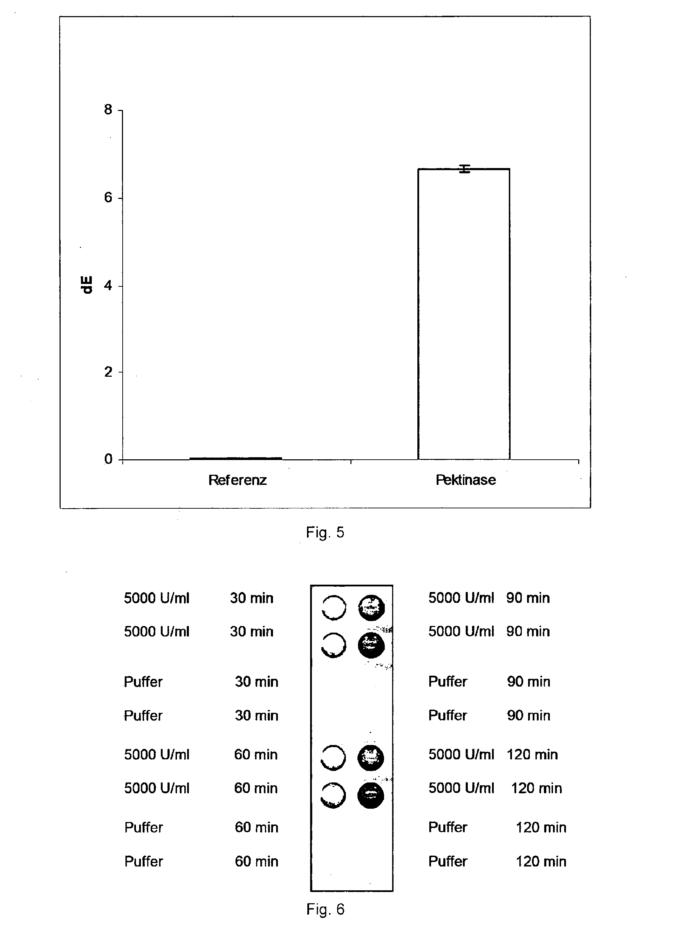

Die Polymerkugeln wurden abgesiebt, 3-mal mit 50mM Succinat Puffer pH4,5 gewaschen und zu je 1g Feuchtmasse in Reaktionsgefäßen mit 5mL 50mM Succinat Puffer pH4,5 in Anwesenheit von 10mg Kieselgel mit immobilisierter Ferulasäure als Laccasesubstrat aus Beispiel 2 portioniert. Der enzymatische Abbau wurde durch Zugabe kommerziell erhältlicher Pektinase bei Raumtemperatur und unter Schütteln gestartet. Nach der Inkubation wurde der Farbumschlag zu gelb-orange anhand einer Farbmessung mittels Spectrophotometer bestimmt (Fig. 5).

Alternativ dazu kann das Biopolymer mit einer Protease von Aspergillus oryzae beladen werden. Hier wird dann entsprechend die Aktivität der freigesetzten Protease mittels UV-Absorption des abgespaltenen p-Nitroanilides im Überstand bei 375 nm bestimmt.

### Beispiel 9:

### Enzymatische Signalgebung und deren Verstärkung durch Freisetzung von modifizierten Proteasen mittels Pektinasen

Das Ansprechverhalten der bioresponsiven Polymere kann über das Diffusionsverhalten sowohl der Trigger- als auch der Verstärkerenzyme eingestellt werden. Durch eine chemische oder genetische Modifikation (z.B. Vergrößerung) der Verstärkerenzyme können entsprechend geringere Vernetzungsgrade des Biopolymers eingesetzt werden, bei einer gleichzeitigen Minimierung der Ausdiffusion des Verstärkerenzyms.

Zur chemischen Modifikation von Verstärkerenzymen mit wasserlöslichen Polymeren wurden 1g Methoxypolyethylenglykol und 0,4g Cuyanurchlorid in 100mL trockenem Toluol gelöst und für 40 Stunden bei 40°C gerührt. Anschließend wurde das aktivierte Polymer in Hexan gefällt, filtriert und unter Vakuum getrocknet. Durch Verwendung unterschiedlicher Molekulargewichte (350, 550, 1100, 2000, 5000 etc.) kann mittels der Länge des Polymers das Diffusionsverhalten des Konjugates eingestellt werden. Das Polymer wurde in schwach basischem Medium Boratpuffer pH9,3 an das Enzym gehängt. Nach der Reaktion wurde ungebundenes Polymer mittels Ultrafiltration entfernt und das Konjugat ohne weitere Aufreinigung verwendet.

Entsprechend Beispiel 6 wurde das Biopolymer mit modifizierter Protease beladen. Die so gewonnenen Polymerkugeln wurden abgesiebt, 3-mal mit 50mM Succinat Puffer pH4,5 gewaschen und zu je 1g Feuchtmasse in Reaktionsgefäßen mit 5mL 50mM Succinat Puffer pH4,5 in Anwesenheit von 10mg Kieselgel mit immobilisierter Ferulasäure als Laccasesubstrat aus Beispiel 2 portioniert. Der enzymatische Abbau bei Raumtemperatur und unter Schütteln wurde durch Zugabe kommerzieller Pektinase gestartet. Nach der Inkubation wurde der Farbumschlag zu gelb-orange anhand einer Farbmessung mittels Spectrophotometer bestimmt (Fig. 5). Alternativ hierzu kann das Molekulargewicht des Triggerenzyms gentechnisch erhöht werden.

### Beispiel 10:

### Enzymatische Signalgebung und deren Verstärkung durch Freisetzung von modifizierter Laccase mittels Lysozym

Ein System das im medizinischen Bereich zur Anwendung gebracht werden kann benützt das Enzym Lysozym als Trigger Enzym. Dieses Enzym der körpereigenen, nativen Immunantwort wird im Falle von Infektion gebildet und sekretiert. Hauptaufgabe des Enzyms ist eine Zerstörung von Bakterien durch Abbau von Peptidoglycan, einem Bestandteil der bakteriellen Zellwand. Es wurde gezeigt, dass erhöhte Enzymmengen im Falle einer Wundinfektion vorliegen. Im folgenden System wurden 3,12mg Mikrokokkus lysodeicticus Zellwand von Sigma mit 1mL 1%iger Agarose in Phosphat-Puffer pH7,00 suspendiert. 100µl dieser Suspension wurden in einer Mikrotiterplatte mit 50µL einer PEG modifizierten Laccase vermischt. Nach Aushärten wird das Polymer mit Puffer gewaschen und 100µl einer Lysozymlösung (200-5000U/mL) aufgetragen. Die Inkubation erfolgte bei 37°C. Alle 30 Minuten wurden 25µl des Überstandes entnommen. Laccaseaktivität wurde mittels des ABTS Assays nachgewiesen (1400µL Saccharose Puffer + 45µL 1% H₂O₂ + 30µL ABTS 40mM): 25µL Überstand + 75µL ABTS-Lösung). Eine Grünfärbung trat nach einigen Minuten Inkubation mit Lysozym durch Umsetzung von ABTS durch die freigesetzte Laccase auf (Fig. 6).

### Beispiel 11:

### Quervernetztes Biopolymer und Freisetzung von Enzymen mittels Pektinasen oder Cellulasen

5 g Pektine oder/und Cellulosederivate werden in 100 mL Wasser gelöst und mit 3 3mL einer 97% Glycidylmethacrylate Lösung in Gegenwart von 0,5 mL 6 M HCl gekoppelt.

PES Gewebe wird mit Trimethylsilyl methacrylate modifiziert und mit einer mit Glycidylmethacrylate modifizierten Matrix überschichtet und radikalisch auspolymerisiert und damit kovalent quervernetzt.

Extrazellulare Enzyme wie Pektinasen oder Cellulasen können nun das PES Geweben durch Diffusion überwinden und durch Abbau der Matrix darin eingeschlossene funktionelle Moleküle wie z.B. Enzyme freisetzen, die ihrerseits eine Farbreaktion auslösen.

### Beispiel 12:

### Quervernetztes Biopolymer und Freisetzung von Enzymen durch Freisetzung von modifizierten Enzymen mittels Elastase

Ein weiteres System, das im medizinischen Bereich zur Anwendung gebracht werden kann, benützt das Enzym Elastase als Trigger Enzym. Dieses Enzym wird von manchen Bakterienarten, aber auch von der körpereigenen Immunantwort im Falle von Infektion gebildet und sekretiert, wobei fast alle Arten von Protein gespalten werden können. Im Falle von Wundinfektion konnten eindeutig erhöhte Enzymmmengen im Wundsekret nachgewiesen werden. Damit kann dieses Enzym als Markerenzym für beginnende Wundinfektion herangezogen werden. Im folgenden System wurden 3,12mg Chitosan mit 1mL 1%iger Agarose in Phosphat-Puffer pH 7,0 suspendiert. Das Chitosan wurde zuvor mit GMBS aktiviert, um es beidseits über die SH Gruppe eines Cysteins und der Peptidsequenz Ala-Ala-Pro-Val querzuvernetzen. 100µl dieser Suspension wurden in einer Mikrotiterplatte mit 50µL einer PEG modifizierten Laccase vermischt. Nach Aushärten wird das Polymer mit Puffer gewaschen und 100µl einer Elastaselösung (2-5 U/mL) oder mit Wundsekret aufgetragen. Die Inkubation erfolgte bei 37°C. Alle 10 Minuten wurden 25µl des Überstandes entnommen. Laccaseaktivität wurde mittels des ABTS Assays nachgewiesen (1400µL Saccharose Puffer + 45µL 1% H₂O₂ + 30uL ABTS 40mM) : 25µL Überstand + 75µL ABTS-Lösung). Eine Grünfärbung trat nach einigen Minuten Inkubation mit Elastase durch Umsetzung von ABTS durch die freigesetze Laccase auf. Ohne Elastase wurde keine Farbänderung festgestellt.

## Patentansprüche

1. Anordnung umfassend einen festen Träger und eine an dem festen Träger angeordnete Matrix umfassend mindestens ein enzymatisch umsetzbares oder modifizierbares Molekül, welche Matrix mindestens ein durch Umsetzung oder Modifikation des Moleküls freisetzbares Enzym umfasst, das in der Lage ist zumindest ein in der Matrix und/oder am festen Träger befindliches farbänderndes Substrat umzusetzen.

2. Anordnung nach Anspruch 1, **dadurch gekennzeichnet, dass** die Matrix als Schicht, in Form von Kapseln oder als Hydrogel auf den festen Träger angeordnet ist.

3. Anordnung nach Anspruch 1 oder 2, **dadurch gekennzeichnet, dass** das enzymatisch umsetzbare oder modifizierbare Molekül ein Polymer, vorzugsweise ein Polysaccharid, Polypeptid, Polyester, Polyamid oder eine Kombination davon ist, ist.

4. Anordnung nach Anspruch 3, **dadurch gekennzeichnet, dass** das Polysaccharid ausgewählt aus der Gruppe bestehend aus Pektin, Amylose, Amylopektin, Agarose, Alginat, Carraghenan, Chitin, Chitosan, Dextran, Glycogen, Guar, Johannisbrotkernmehl, Laevan, Pektin, Pollulan, Tamarindenkernmehl, Xanthan und Xylan.

5. Anordnung nach einem der Ansprüche 1 bis 4, **dadurch gekennzeichnet, dass** die Matrix zusätzlich durch Umsetzung oder Modifikation des Moleküls freisetzbares Enzym umfasst, das in der Lage ist das in der Matrix befindliche Molekül ebenfalls umzusetzen oder zu modifizieren.

6. Anordnung nach Anspruch 5, **dadurch gekennzeichnet, dass** das Enzym ausgewählt ist aus der Gruppe bestehend aus den Gruppen der Hydrolasen und Oxidoreduktasen, wie Proteasen, Laccasen oder Peroxidasen.

7. Anordnung nach einem der Ansprüche 1 bis 6, **dadurch gekennzeichnet, dass** das durch die Umsetzung oder Modifikation des Moleküls freisetzbare Enzyme an Polyvinylalkohol, Polyethylenglykol (PEG), Polypeptide, insbesondere Elastin, oder Peptide gebunden ist.

8. Anordnung nach Anspruch 7, **dadurch gekennzeichnet, dass** das freisetzbare Enzym, welches mittels eines Polypeptids oder Peptids am enzymatisch umsetzbaren oder modifizierbaren Molekül gebunden ist, durch ein Enzym, vorzugsweise einem mikrobiellen Enzym oder einem Enzym des Immunsystems, wie Elastase, freisetzbar ist.

9. Anordnung nach einem der Ansprüche 1 bis 8, **dadurch gekennzeichnet, dass** das farberzeugende Substrat ausgewählt ist aus der Gruppe bestehend aus phenolischen Verbindungen und Azo-Farbstoffen.

10. Anordnung nach Anspruch 9, **dadurch gekennzeichnet, dass** das farberzeugende Substrat direkt von Enzymen, vorzugsweise mikrobiellen Enzymen, wie Laccasen oder Peroxidasen oder Enzymen des Immunsystems, wie Myeloperoxidase, umsetzbar ist.

11. Anordnung nach einem der Ansprüche 1 bis 10, **dadurch gekennzeichnet, dass** die Anordnung eine dem festen Träger gegenüberliegende semipermeable Membran aufweist, wobei die semipermeable Membran vorzugsweise ausgewählt ist aus der Gruppe bestehend aus Cellulosederivate, Polyamide, Polyacrylamide und Polyester.

12. Anordnung nach einem der Ansprüche 3 bis 11, **dadurch gekennzeichnet, dass** das Polymer, das Enzym und das farbändernde Substrat ausgewählt sind aus Tabelle A.

13. Verwendung einer Anordnung nach einem der Ansprüche 1 bis 12 zur Bestimmung der Anwesenheit und/oder Charakterisierung von Zellen, vorzugsweise von Mikroorganismen, vorzugsweise ausgewählt aus der Gruppe bestehend aus Bakterien und Pilzen, in einer Probe.

14. Verwendung einer Anordnung nach einem der Ansprüche 1 bis 12 zur Detektion mindestens eines Enzyms, vorzugsweise ausgewählt aus der Gruppe der Hydrolasen bestehend aus Amylase, Cellulase, Xylanase, Mannanase, Protease, Lysozym, Elastase, Collagenase, Cathepsin, Myeloperoxidase, Lipase und Esterase, in einer Probe.

15. Verwendung einer Anordnung nach einem der Ansprüche 1 bis 12 zur Detektion einer Wundinfektion durch Bestimmen des Vorhandenseins mindestens eines wund-spezifischen Enzyms, vorzugsweise aus der Gruppe der Hydrolasen bestehend aus Amylase, Cellulase, Xylanase, Mannanase, Protease, Lysozym, Elastase, Collagenase, Cathepsin, Myeloperoxidase, Lipase und Esterase.

## Claims

1. An arrangement comprising a solid carrier and a matrix arranged on the solid carrier, comprising at least one enzymatically convertible or modifiable molecule, which matrix comprises at least one enzyme that can be released by conversion or modification of the molecule, preferably polymer or oligomer, which enzyme is capable of converting at least one colour-changing substrate located in the matrix and/or on the solid carrier.

2. The arrangement according to claim 1, **characterised in that** the matrix is arranged as a layer, in the form of capsules or as a hydrogel on the solid carrier.

3. The arrangement according to claim 1 or 2, **characterised in that** the enzymatically convertible or modifiable molecule is a polymer, preferably a polysaccharide, polypeptide, polyester, polyamide or a combination thereof.

4. The arrangement according to claim 3, **characterised in that** the polysaccharide is selected from the group consisting of pectin, amylose, amylopectin, agarose, alginate, carrageenan, chitin, chitosan, dextran, glycogen, guar, locust bean gum flour, laevan, pectin, pollulan, tamarind seed flour, xanthan and xylan.

5. The arrangement according to any one of claims 1 to 4, **characterised in that** the matrix additionally comprises an enzyme that can be released by conversion or modification of the molecule that is capable of likewise converting or modifying the molecule located in the matrix.

6. The arrangement according to claim 5, **characterised in that** the enzyme is selected from the group consisting of the groups of hydrolases and oxidoreductases such as proteases, laccases or peroxidases.

7. The arrangement according to any one of claims 1 to 6, **characterised in that** the enzyme that can be released by conversion or modification of the molecule is bound to polyvinyl alcohol, polyethylene glycol (PEG), polypeptide, in particular elastin or peptides.

8. The arrangement according to claim 7, **characterised in that** the enzyme which can be released, which is bound by means of a polypeptide or peptide to the enzymatically convertible or modifiable molecule, can be released by an enzyme, preferably a microbial enzyme or an enzyme of the immune system such as elastase.

9. The arrangement according to any one of claims 1 to 8, **characterised in that** the colour-producing substrate is selected from the group consisting of phenolic compounds and azo dyes.

10. The arrangement according to claim 9, **characterised in that** that the colour-producing substrate can be converted directly by enzymes, preferably microbial enzymes such as laccases or peroxidases or enzymes of the immune system such as myeloperoxidase.

11. The arrangement according to any one of claims 1 to 10, **characterised in that** the arrangement comprises a semi-permeable membrane opposite the solid carrier, wherein the semi-permeable membrane is selected from the group consisting of cellulose derivatives, polyamides, polyacrylamides and polyester.

12. The arrangement according to any one of claims 3 to 11, **characterised in that** the polymer, the enzyme and the colour-changing substrate are selected from Table A.

13. Use of an arrangement according to any one of claims 1 to 12 to determine the presence and/or characterisation of cells, preferably microorganisms, preferably selected from the group consisting of bacteria and fungi, in a sample.

14. The use of an arrangement according to any one of claims 1 to 12 for the detection of at least one enzyme, preferably selected from the group of hydrolases consisting of amylase, cellulase, xylanase, mannanase, protease, lysozyme, elastase, collagenase, cathepsin, myeloperoxidase, lipase and esterase in a sample.

15. The use of an arrangement according to any one of claims 1 to 12 for the detection of a wound infection by determining the presence of at least one wound-specific enzyme, preferably from the group of hydrolases consisting of amylase, cellulase, xylanase, mannanase, protease, lysozyme, elastase, collagenase, cathepsin, myeloperoxidase, lipase and esterase.

## Revendications

1. Arrangement comprenant un support solide et une matrice disposée contre le support solide, comprenant au moins une molécule pouvant être convertie ou modifiée par voie enzymatique, laquelle matrice comprend au moins une enzyme pouvant être libérée par conversion ou modification de la molécule, enzyme qui est à même de convertir au moins un substrat modificateur de couleur, se trouvant dans la matrice et/ou contre le support solide.

2. Arrangement selon la revendication 1, **caractérisé en ce que** la matrice est disposée sur le support solide sous forme d'une couche, sous forme de capsules ou sous forme d'un hydrogel.

3. Arrangement selon la revendication 1 ou 2, **caractérisé en ce que** la molécule pouvant être convertie ou modifiée par voie enzymatique est un polymère, de préférence un polysaccharide, un polypeptide, un polyester, un polyamide ou une combinaison de ceux-ci.

4. Arrangement selon la revendication 3, **caractérisé en ce que** le polysaccharide est choisi dans le groupe consistant en la pectine, l'amylose, l'amylopectine, l'agarose, l'alginate, le carragénane, la chitine, le chitosan, le dextran, le glycogène, la gomme de guar, la gomme de caroube, le lévane, la pectine, le pollulane, la farine de tamarin, le xanthane et le xylane.

5. Arrangement selon l'une des revendications 1 à 4, **caractérisé en ce que** la molécule comprend en outre une enzyme pouvant être libérée par conversion ou modification de la molécule, enzyme qui est à même de convertir ou de modifier aussi la molécule se trouvant dans la matrice.

6. Arrangement selon la revendication 5, **caractérisé en ce que** l'enzyme est choisie dans le groupe consistant en les groupes des hydrolases et des oxydoréductases, telles que les protéases, les laccases ou les peroxydases.

7. Arrangement selon l'une des revendications 1 à 6, **caractérisé en ce que** l'enzyme pouvant être libérée par conversion ou modification de la molécule est liée à un poly(alcool vinylique), à un polyéthylèneglycol (PEG), à des polypeptides, en particulier l'élastine, ou à des peptides.

8. Arrangement selon la revendication 7, **caractérisé en ce que** l'enzyme pouvant être libérée, qui est liée par l'intermédiaire d'un polypeptide ou d'un peptide à la molécule pouvant être convertie ou modifiée par voie enzymatique, peut être libérée par une enzyme, de préférence une enzyme microbienne ou une enzyme du système immunitaire, telle que l'élastase.

9. Arrangement selon l'une des revendications 1 à 8, **caractérisé en ce que** le substrat chromogène est choisi dans le groupe consistant en les composés phénoliques et les colorants azoïques.

10. Arrangement selon la revendication 9, **caractérisé en ce que** le substrat chromogène peut être converti directement par des enzymes, de préférence des enzymes microbiennes telles que les laccases ou les peroxydases, ou des enzymes du système immunitaire, telles que la myéloperoxydase.

11. Arrangement selon l'une des revendications 1 à 10, **caractérisé en ce que** l'arrangement présente une membrane semi-perméable opposée au support solide, la membrane semi-perméable étant de préférence choisie dans le groupe consistant en les dérivés de la cellulose, les polyamides, les polyacrylamides et les polyesters.

12. Arrangement selon l'une des revendications 3 à 11, **caractérisé en ce que** le polymère, l'enzyme et le substrat modificateur de couleur sont choisis dans le Tableau A.

13. Utilisation d'un arrangement selon l'une des revendications 1 à 12 pour déterminer dans un échantillon la présence et/ou la caractérisation des cellules, de préférence de microorganismes, de préférence choisies dans le groupe consistant en les bactéries et les champignons.

14. Utilisation, dans un échantillon, d'un arrangement selon l'une des revendications 1 à 12 pour la détection d'au moins une enzyme, de préférence choisie dans le groupes des hydrolases consistant en une amylase, une cellulase, une xylanase, une mannanase, une protéase, un lysozyme, une élastase, une collagénase, une cathepsine, une myéloperoxydase, une lipase et une estérase.

15. Utilisation d'un arrangement selon l'une des revendications 1 à 12 pour la détection de l'infection d'une plaie par détermination de la présence d'au moins une enzyme spécifique de plaie, de préférence du groupe des hydrolases, consistant en une amylase, une cellulase, une xylanase, une mannanase, une protéase, un lysozyme, une élastase, une collagénase, une cathepsine, une myéloperoxydase, une lipase et une estérase.
